# EUROPEAN PATENT APPLICATION

(11) **EP 1 783 125 A1**
(43) Date of publication of application: **09.05.2007**
(21) Application number: 05780394.2
(22) Date of filing: 11.08.2005
(51) Int. Cl.: C07D 401/04

(54) **METHOD FOR PRODUCING 2-(4-METHYL-2-PHENYLPIPERAZINE-1-YL)-3-CYANOPIRIDINE**

(30) Priority: 24.08.2004 JP 2004243419; 22.04.2005 JP 2005124722
(71) Applicant: Sumitomo Chemical Company, Limited, Tokyo 104-8260 (JP)
(72) Inventor: YAMAMOTO, Tomiaki, 5008082 (JP); INOUE, Haruyuki, 5010521 (JP); SHIMOKAWA, Kaoru, 5030126 (JP)
(74) Representative: Winter, Brandl, Fürniss, Hübner Röss, Kaiser, Polte Partnerschaft Patent- und Rechtsanwaltskanzlei
(86) International application number: PCT/JP2005/015038
(87) International publication number: WO 2006/022182

(57) **Abstract**

The present invention provides a process for producing 2-(4-methyl-2-phenylpiperazin-1-yl)-3-cyanopyridine which comprises reacting 1-methyl-3-phenylpiperazine with 2-chloro-3-cyanopyridine in the presence of an organic base and in the absence of alkali metal halide in an polar aprotic organic solvent.

## Description

### FIELD OF THE INVENTION

The present invention relates to a process for producing 2-(4-methyl-2-phenylpiperazin-1-yl)-3-cyanopyridine; in more detail, relates to a process for producing 2-(4-methyl-2-phenylpiperazin-1-yl)-3-cyanopyridine suitably usable as an intermediate to produce mirtazapine which is useful as an antidepressant.

### BACKGROUND OF THE INVENTION

Mirtazapine is a useful compound as an antidepressant, and 2-(4-methyl-2-phenylpiperazin-1-yl)-3-cyanopyridine is known as an intermediate to produce the mirtazapine. As the method for producing 2-(4-methyl-2-phenylpiperazin-1-yl)-3-cyanopyridine, known is a method of reacting 1-methyl-3-phenylpiperazine with 2-chloro-3-cyanopyridine in the presence of potassium fluoride (JP59-42678-B).

However, this method is not economical because of using expensive potassium fluoride as well as has industrial disadvantages that an apparatus using a glass or being with a glass lining is not applicable for production due to corrosion, and that it is hard to take up 2-(4-methyl-2-phenylpiperazin-1-yl)-3-cyanopyridine as an objective compound from a reaction solution due to a presence of a large amount of by-produced tar.

As alternative, known is a method of producing 2-(4-methyl-2-phenylpiperazin-1-yl)-3-cyanopyridine by using potassium iodide in place of potassium fluoride and reacting in the presence of a base in the same manner (WO01/023345A1).

Potassium iodide, however, is relatively expensive in view of industrial production scale, resulting in an economical problem.

### SUMMARY OF THE INVENTION

The present invention, under consideration of prior arts mentioned above, intends to provide a process allowing to industrially easily and economically produce 2-(4-methyl-2-phenylpiperazin-1-yl)-3-cyanopyridine useful as an intermediate to produce mirtazapine without using an expensive potassium fluoride or potassium iodide.

The present inventors have diligently studied to solve the problems mentioned above and achieved the present invention.

That is, the inventions are as follows:
[1] A process for producing 2-(4-methyl-2-phenylpiperazin-1-yl)-3-cyanopyridine which comprises reacting 1-methyl-3-phenylpiperazine with 2-chloro-3-cyanopyridine in the presence of an organic base and in the absence of alkali metal halide in an polar aprotic organic solvent.
[2] The process according to [1], wherein the organic base is an alkylamine.
[3] The process according to [2], wherein the alkylamine is triethylamine.
[4] The process according to any of [1] to [3], wherein the amount of 1-methyl-3-phenyl-piperadine is 0.6 to 1.1 mol per one mol of 2-chloro-3-cyanopyridine.
[5] A process for producing 2-(4-methyl-2-phenylpiperadin-1-yl)-3-cyanopyridine, which comprises mixing 1-methyl-3-phenylpiperadine, 2-chloro-3-cyanopyridine and an organic base, and optionally a quaternary ammonium in a polar aprotic organic solvent.

### DESCRIPTION OF PREFERRED EMBODIMENTS

The production of 2-(4-methyl-2-phenylpiperazin-1-yl)-3-cyanopyridine can be easily carried out by reacting 1-methyl-3-phenylpiperazine with 2-chloro-3-cyanopyridine in the presence of an organic base and in the absence of alkali metal halide in a polar aprotic organic solvent.

1-Methyl-3-phenylpiperazine can be produced, for example, by a method disclosed in WO01/023345. 2-Chloro-3-cyanopyridine is commercially available.

The amount of 1-methyl-3-phenylpiperazine is usually 0.6 to 1.1 mols per 1 mol of 2-chloro-3-cyanopyridine in view of sufficient progress of reaction with 2-chloro-3-cyanopyridine, and preferably 0.65 to 0.9 mols.

Examples of the organic base include alkylamines such as triethylamine, diisopropylethylamine, and the like; cyclic amines such as N-methylmorpholine, and the like; aromatic amines such as pyridine, picoline, and the like; and the like. Among them, triethylamine is preferred in view of economics. The amount of the organic base, in view of sufficient progress of reaction of 1-methyl-3-phenylpiperazine with 2-chloro-3-cyanopyridine and of economics, is usually 1.1 to 2 mols per 1 mol of 1-methyl-3-phenylpiperazine, and preferably 1.3 to 1.5 mols.

Examples of the polar aprotic organic solvent include dimethylformamide, dimethylacetamide, dimethylsulfoxide, and 1,3-dimethylimidazolidin-2-one, and the like. Among them, dimethylformamide is preferably usable in view of economics. The amount of the solvent is not particularly limited, but is usually 100 to 500 parts by volume per 100 parts by weight of 1-methyl-3-phenylpiperazine, and preferably 150 to 400 parts by volume.

In the present invention, a quaternary ammonium salt, for example, such as tetrabutylammonium iodide, tetrabutylammonium bromide, benzyltrimethylammonium chloride may be used in an appropriate amount as a catalyst.

The reaction of 1-methyl-3-phenylpiperazine with 2-chloro-3-cyanopyridine is preferably carried out under an inert gas such as nitrogen, argon, and the like. Specifically, the reaction may be carried out, for example, by mixing 1-methyl-3-phenylpiperazine, 2-chloro-3-cyanopyridine, and the organic base, and if necessary, further a quaternary ammonium salt, in the polar aprotic organic solvent under the inert gas atmosphere mentioned above.

The reaction temperature is usually 90 to 160 °C, preferably 110 to 150 °C, and more preferably 110 to 130 °C, in view of enhancing the reaction rate and suppressing formation of by-products. The reaction time varies depending on the reaction temperature, and can not be unable to fix in a certain value, and is usually from about 12 to 30 hours.

After finishing the reaction of 1-methyl-3-phenylpiperazine with 2-chloro-3-cyanopyridine, 2-(4-methyl-2-phenylpiperazin-1-yl)-3-cyanopyridine can easily be isolated by concentrating solvent contained in the reaction solution or by adding water, extracting with solvent such as ethyl acetate, and concentrating to obtain a crude product; or by re-crystallizing from an appropriate solvent.

For example, the reaction liquid is treated as follows: distilling out 75 to 95 % of dimethylformamide used for the reaction at an inner temperature of 70 to 95 °C under a reduced pressure of 7 to 2.7 kPa, and being added with 100 to 250 parts by weight of water per 100 parts by weight of 1-methyl-3-phenylpiperazine at from 70 to 80 °C.

Thereafter, pH value thereof is adjusted to 8 to 9 with an alkali. Examples of the alkali include sodium hydroxide, sodium carbonate, and the like. When sodium hydroxide is used as the alkali, it may be usually used in a form of 10 to 40 % by weight aqueous sodium hydroxide solution.

Thereafter, this reaction solution is extracted with a solvent such as ethyl acetate, and the like. When ethyl acetate is used as the solvent, it is usually used in an amount of 300 to 800 parts by weight based on 100 parts by weight of 1-methyl-3-phenylpiperazine. The extraction temperature is preferably at from 40 to 50 °C.

Furthermore, in the present invention, 2-(4-methyl-2-phenylpiperazin-1-yl)-3-cyanopyridine may be isolated in a form of a salt thereof by dissolving produced 2-(4-methyl-2-phenylpiperazin-1-yl)-3-cyanopyridine in an organic solvent such as ethyl acetate, methanol, ethanol, and the like, adding an acid thereto, collecting crystals by filtration, and then drying the crystals. In this case, as the acid, for example, organic acids such as oxalic acid, succinic acid, maleic acid, methanesulfonic acid, toluenesulfonic acid, and the like, and inorganic acids such as sulfuric acid, hydrochloric acid, phosphoric acid, and the like, may be used. Among them, oxalic acid is preferred in view of crystallinity, purity, and yield.

For example, a solution containing 2-(4-methyl-2-phenylpiperazin-1-yl)-3-cyanopyridine which is extracted from the reactant solution, may be added with 100 to 150 parts by weight of methanol per 100 parts by weight of 1-methyl-3-phenylpiperazine and then further added with oxalic acid dihydrate at 40 to 50 °C, or added dropwise with a solution in which oxalic acid is dissolved in methanol in a ratio of 250 to 400 parts by weight of methanol per 100 parts by weight of oxalic acid. Oxalic acid is preferably in an amount of 0.9 to 1.5 mole per 1 mol of 1-methyl-3-phenylpiperazine.

Thereafter, this solution is cooled down to 15 to 25 °C, matured for 1 to 10 hours, and then filtrated to collect, and then optionally washed with a mixed solution of methanol and ethyl acetate (for example, 3 to 4 parts by volume of ethyl acetate per 1 part by volume of methanol); thereafter, the collected crystals were dried at a temperature of 50 to 60 °C to obtain 2-(4-methyl-2-phenylpiperazin-1-yl)-3-cyanopyridine oxalate.

Thus obtained 2-(4-methyl-2-phenylpiperazin-1-yl)-3-cyanopyridine or an oxalic acid salt thereof is a useful compound as an intermediate to produce mirtazapine. According to the present invention, 2-(4-methyl-2-phenylpiperazin-1-yl)-3-cyanopyridine can be effectively obtained without using alkali metal halide, the alkali metal halide conventionally being an inevitable component for producing 2-(4-methyl-2-phenylpiperazin-1-yl)-3-cyanopyridine by reacting 1-methyl-3-phenylpiperazine with 2-chloro-3-cyanopyridine; furthermore, the process of the present invention is expected to have an enhanced yield of the objective product in comparison with the conventional method mentioned above.

The invention will be explained in more detail based on Examples, but should not be construed thereto.

### Example 1

### 2-(4-methyl-2-phenylpiperazin-1-yl)-3-cyanopyridine oxalate

After adding 21.1 g (119.7 mmol) of 1-methyl-3-phenylpiperazine, 20.0 g (144.4 mmol) of 2-chloro-3-cyanopyridine, and 16.6 g (164.1 mmol) of triethylamine to 42 g of dimethylformamide, the mixture was reacted at 125 to 130 °C for 24 hours under a nitrogen atmosphere. After distilling out triethylamine and dimethylformamide from the reactant solution under a reduced pressure, the residue was added with 32 ml of water and 87 g of ethyl acetate, and then a pH value thereof was adjusted to 8 to 9 with 10 % aqueous sodium hydroxide solution. After phase-separating the solution, an organic layer was added with 24 g of methanol, and then 15.2 g of oxalic acid. This solution was filtrated to collect crystals, and then the crystals collected were dried to obtain 31.6 g of an objective compound (HPLC content : 86.1 %, the yield from 1-methyl-3-phenylpiperazine was 61.7 %). IR (KBr) γ=3039, 2223, 1733, 1636, 1578, 1567, 1436, 758, 701 cm⁻¹1H-NMR (CDC13, 400 MHz) δ ppm: 8.29, 7.77, 6.76 (dd, each 1H); 7.1-7.44 (m, 5H); 5.46 (t, 1H, CHPh); 3.83, 3.59 (m, each H); 2.95 (dd, 1H); 2.65-2.80 (m, 4H); 2.25 (m, 1H); 2.33 (s, 3H, NCH3).

### Example 2

### 2-(4-methyl-2-phenylpiperazin-1-yl)-3-cyanopyridine oxalate

After adding 21.1 g (119.7 mmol) of 1-methyl-3-phenylpiperazine, 24.0 g (173.2 mmol) of 2-chloro-3-cyanopyridine, and 16.6 g (164.1 mmol) of triethylamine to 42 g of dimethylformamide, the mixture was reacted at 125 to 130 °C for 24 hours under a nitrogen atmosphere. After distilling out triethylamine and dimethylformamide from the reactant solution under a reduced pressure, the solution was added with 32 ml of water and 87 g of ethyl acetate, and then a pH value thereof was adjusted to 8 to 9 with 10 % aqueous sodium hydroxide solution. After phase-separating the solution, an organic layer was added with 24 g of methanol, and then 15.2 g of oxalic acid. This solution was filtrated to collect crystals, and then the crystals collected were dried to obtain 31.9 g of an objective compound (HPLC content : 92.4 %, the yield from 1-methyl-3-phenylpiperazine was 66.8 %).

### Example 3

### 2-(4-methyl-2-phenylpiperazin-1-yl)-3-cyanopyridine oxalate

In 57.3 kg of dimethylformamide solution containing 21.3 kg of 1-methyl-3-phenylpiperazine, 22.2 kg of 2-chloro-3-cyanopyridine and 15.3 kg of triethylamine were added, the mixture was reacted at 114 to 125 °C for 17 hours under a nitrogen atmosphere. The reaction solution was concentrated under a reduced pressure. The distillated amount was 36 kg. The residue was added with 29.3 kg of water and then a pH value thereof was adjusted to 8.45 with 25 % aqueous sodium hydroxide solution. This solution was added with 79.2 kg of ethyl acetate, washed with 20 kg of 5 % sodium chloride solution, and then subjected to a phase separation. An organic layer was added with 23.1 kg of methanol, and then added with 13.9 kg of oxalic acid dihydrate at a temperature of 45 to 48 °C for about 1 hour. The solution was stirred at the temperature for 1 hour, filtrated at around 35 °C to collect crystals, and then the crystals collected were washed with a mixture of 42.2 kg of ethyl acetate and 12.4 kg of methanol. The crystals were dried at around 50 °C under a reduced pressure to obtain 32.65 kg of an objective compound (HPLC content : 90.2 %, the yield from 1-methyl-3-phenylpiperazine was 66.2 %).

### Comparative Example 1

After adding 21.1 g (119.7 mmol) of 1-methyl-3-phenylpiperazine, 20.0 g (144.4 mmol) of 2-chloro-3-cyanopyridine, 12.8 g (126.3 mmol) of triethylamine, and 2.0 g (12.0 mmol) of potassium iodide to 42 g of dimethylformamide, the mixture was reacted at 125 to 130 °C for 24 hours under a nitrogen atmosphere. After distilling out triethylamine and dimethylformamide from the reactant solution under a reduced pressure, the residue was added with 32 ml of water and 87 g of ethyl acetate, and then a pH value thereof was adjusted to 8 to 9 with 10 % aqueous sodium hydroxide solution. After phase-separating the solution, an organic layer was added with 24 g of methanol, and then 15.2 g of oxalic acid. This solution was filtrated to collect crystals, and then the crystals collected were dried to obtain 26.6 g of 2-(4-methyl-2-phenylpiperazin-1-yl)-3-cyanopyridine oxalate (HPLC content : 93.8 %, the yield from 1-methyl-3-phenylpiperazine was 56.6 %).

### Comparative Example 2

In 86.2 g of dimethylformamide solution containing 32.1 g of 1-methyl-3-phenylpiperazine, 36.3 g of 2-chloro-3-cyanopyridine was added, the mixture was reacted at 120 to 125 °C for 18 hours under a nitrogen atmosphere. The reaction solution was concentrated under a reduced pressure. The solution was subjected to after-treatments in the same manner as in Example 3, and then measured with the HPLC. 2-(4-Methyl-2-phenylpiperazin-1-yl)-3-cyanopyridine was generated in 40.9 % yield based on 1-methyl-3-phenylpiperazine.

### HPLC measurement conditions:

Column : ODS column (SUMIPAX ODS A-212)
Mobile phase : A solution A solution of dissolving 0.05 mole of disodium hydrogenphosphate in 1 liter of purified water, of which pH value was adjusted to 6 with phosphoric acid
B solution Acetonitrile
A solution : B solution = 55 : 45
Detection wavelength : UV220 nm

According to the process of the present invention, 2-(4-methyl-2-phenylpiperazin-1-yl)-3-cyanopyridine useful as an intermediate of mirtazapine can be obtained more industrially advantageously than the conventional methods.

## Claims

1. A process for producing 2-(4-methyl-2-phenylpiperazin-1-yl)-3-cyanopyridine which comprises reacting 1-methyl-3-phenylpiperazine with 2-chloro-3-cyanopyridine in the presence of an organic base and in the absence of alkali metal halide in an polar aprotic organic solvent.

2. The process according to Claim 1, wherein the organic base is an alkylamine.

3. The process according to Claim 2, wherein the alkylamine is triethylamine.

4. The process according to Claim 1, wherein the amount of 1-methyl-3-phenyl-piperadine is 0.6 to 1.1 mol per one mol of 2-chloro-3-cyanopyridine.

5. A process for producing 2-(4-methyl-2-phenylpiperadin-1-yl)-3-cyanopyridine, which comprises mixing 1-methyl-3-phenylpiperadine, 2-chloro-3-cyanopyridine and an organic base, and optionally a quaternary ammonium in a polar aprotic organic solvent.
